# EUROPÄISCHE PATENTSCHRIFT

(11) **EP 0 339 054 B1**
(45) Veröffentlichungstag und Bekanntmachung des Hinweises auf die Patenterteilung: **06.07.1994**
(21) Anmeldenummer: 88908106.3
(22) Anmeldetag: 07.10.1988
(51) Int. Cl.: A61K 31/35, A61K 35/78, A61K 31/52, A61K 31/515

(54) **STOFFGEMISCH ZUR STABILISIERUNG DER STOFFWECHSEL-LAGE**
MIXTURE OF SUBSTANCES FOR STABILYZING METABOLISM
MELANGE DE SUBSTANCES POUR STABILISER LE METABOLISME

(30) Priorität: 08.10.1987 AT 2558/87; 25.02.1988 AT 478/88
(43) Veröffentlichungstag der Anmeldung: 02.11.1989
(73) Patentinhaber: Bilas, Andor, A-1070 Wien VII (AT)
(72) Erfinder: Bilas, Andor, A-1070 Wien VII (AT)
(86) Internationale Anmeldenummer: AT8800079
(87) Internationale Veröffentlichungsnummer: WO8903213

(56) Entgegenhaltungen:
- ROTE LISTE, 1863, Editio Cantor-Aulendorf/Württ.; pp. 249, 858, 1018#
- DICTIONNAIRE VIDAL, 1961, O.V.P., Paris (FR); p. 925#

## Beschreibung

Stoffwechselerkrankungen sind in der heutigen Zeit aufgrund der ungesunden Ernährungsgewohnheiten sehr häufig, wobei bekannt ist, daß die einzelnen Stoffwechselkreisläufe beim Abbau der einzelnen Nahrungsmittelbestandteile über entsprechende Seitenwege miteinander verknüpft sind. Es kann daher vorkommen, daß beispielsweise überschüssige Kohlenhydrate in den Fettabbau eingeschleust werden, wo sie dann zur Bildung von Depotfett beitragen.

Manche Stoffwechselerkrankungen, wie z. B. der Typ I Diabetes, sind auf Schädigungen des Immunsystems zurückzuführen, wobei der genannte Typ I Diabetes eine sogenannte "Autoimmunerkrankung" darstellt. Bei diesem Typ I Diabetes bilden sich nämlich Antikörper (Inselzellantikörper) gegen körpereigenes Gewebe, und zwar gegen die insulinproduzierenden Betazellen der Bauchspeicheldrüse, wobei es zu einer Selbstzerstörung dieser Betazellen kommt. Dadurch wird kein Insulin mehr an das Blut ausgeschüttet, so daß dieser Insulinmangel bei dem Typ I Diabetes durch Substitution mittels körperfremden Insulins behandelt wird.

Bei einem Nicht-Diabetiker erfolgt die Regulierung des Glukosehaushaltes durch das Wechselspiel zwischen Insulin- und Glukagon-Ausschüttung. Insulin wird dann ausgeschüttet, wenn durch Nahrungsaufnahme der Blutzuckerspiegel im Körper steigt. Durch Ausschüttung von Insulin wird in einem solchen Fall der Blutzuckerspiegel wieder auf die Normalwerte gesenkt. Sinkt der Blutzuckerspiegel unter ein gewisses Minimum, wird von den Zellen der Bauchspeicheldrüse Glukagon ausgeschüttet, wodurch Glukose aus der Leber an den Blutkreislauf abgegeben und so der Blutzuckerspiegel wieder stabilisiert wird.

Bei Diabetikern ist dieses Wechselspiel Insulinausschüttung/Glukagonausschüttung mehr oder minder stark gestört. Dabei ist dieses Wechselspiel aber nötig, um einerseits eine Überzuckerung (HYPER-Glykämie) zu vermeiden, die bei längerem Andauern u.a. zu einem Verlegen der Nierenmembran und von Kapillargefäßen führen würde, andererseits aber um eine Unterzuckerung (HYPO-Glykämie) zu verhindern, welche schon bei kürzerer Dauer u.a. zu einen Schädigung des Gehirnes und der Augen (Netzhautblutungen, im Extremfall Erblinden) führen würde.

Die Gefahr bei Personen, welche an Diabetes Mellitus, insbesondere vom Typ I leiden, wird nicht nur durch Insulinmangel verursacht, sondern liegt vor allem in den Schäden, welche meistens nach 8 - 13 Jahre dauernder Diabetesbehandlung dadurch einsetzen, daß durch eine Störung der Stoffwechselregelkreise eine mangelhafte Durchblutung,insbesondere der Kapillargefäße, auftritt, die nicht nur Anlaß für Schädigungen sowohl des vegetativen als auch des motorischen Nervensystems ist (diabetische Neuropathie), sondern auch Organe und Blutgefäße in Mitleidenschaft zieht. Es kann dabei u.a. zu folgenden Störungen kommen: Schlafstörungen, Störungen der Schmerz- und Temperaturempfindung, vegetative Dystonie, Störungen der Herzfunktion, Herzinfarkt, Störungen der Nierentätigkeit, Störungen der Darmtätigkeit, Störungen der Magentätigkeit, Störungen der Insulin- und Glukagonproduktion (Schockhypos), Störungen der Gehirntätigkeit, wodurch Störungen der Konzentrationsfähigkeit und des Gedächtnisses auftreten, wobei alle Wahrnehmungen stark beeinträchtigt, verringert und verzögert werden bzw. ganz ausfallen. Der Diabetiker will denken, kann es aber nicht. Versucht er mit Macht, dagegen anzukämpfen, so erlebt er seine eigene Hilflosigkeit. Er fällt Entscheidungen im Glauben, das Richtige zu tun, kann aber die Konsequenzen daraus nicht erfassen. Weiters sind Störungen im Auge (Blutungen in der Netzhaut, Glaskörpertrübungen bis zur Erblindung etc.) sowie Durchblutungsstörungen in den Armen, Beinen, Händen und Füßen, Gangräne, schwer heilende Wunden etc. als Langzeitschäden zu nennen.

Praktisch sind bei einem Langzeit-Diabetiker alle Regelkreise des Körpers empfindlich gestört.

Zusätzlich tritt mit zunehmender Dauer der Krankheit eine Anti-Insulin-Körperreaktion auf. Das Fremd-Insulin wird nämlich vom Körper nicht mehr in gleicher Weise angenommen wie in den ersten Jahren. Das führt zu steigendem Insulinbedarf, wodurch wieder das Auftreten von Langzeitschäden beschleunigt wird. In Rote Liste 1963 wird ein Kombinations präparat enthaltend Khellin und Phenylethylbarbitursäure beschrieben (Coropar®)).

Der Erfindung liegt die Aufgabe zugrunde, den Stoffwechsel und/oder das Immunsystem von Patienten mit Stoffwechselerkrankungen, insbesondere von Diabetikern, zu stabilisieren bzw. zu normalisieren, und zwar dahingehend, daß Langzeitschäden entgegengewirkt wird bzw. bereits aufgetretene Langzeitschäden verbessert werden und/oder ihr Fortschreiten verhindert oder zumindest verzögert wird.

Die Erfindung sieht hierzu die Verwendung mindestens eines Stoffes, ausgewählt aus Phenyläthylbarbitursäure, Papaverin, Belladonnaextrakt und Theobromin im Gemisch mit einem Mittel zur Förderung der peripheren Durchblutung, z.B. Khellin (5,8-Dimethoxy-2-Methylfuro-[4',5':6,7]-Chromon), Khellinabkömmlinge, Pentifylin, Cinnerizin, Xantinolnicotinat oder Extr.Ginkgo bil. bzw. Quercetin (3.5.8.3'.4' Pentaoxy-flavon) und/oder eines Mittels zur Förderung der zentralen Durchblutung, z.B. Erythroltetranitrat, Isosorbitdinitrat, Isosorbitmononitrat, Nitroglycerin, oder Nitropentaerythrolum zur Herstellung eines Arzneimittels zur Stabilisierung bzw. Normalisierung des Stoffwechsels und/oder des Immunsystems von Patienten mit Stoffwechselerkrankungen, wie z.B. von Diabetikern, insbesondere von Insulinpflichtigen, vor. Die vorangeführten Stoffe in der angegebenen Kombination können dabei zur Herstellung eines Arzneimittels gegen Störungen aller Stoffwechselregelkreise und damit auch der peripheren und zentralen Kapillar-Durchblutung sowie zur Verringerung bzw. Vermeidung der Antikörper-Produktion gegen in Diabetes-Mitteln, z.B. Insulin, enthaltenes körperfremdes Eiweiß Verwendung finden.

Durch die erfindungsgemäße Maßnahme kann ein Arzneimittel geschaffen werden, das bei Verabreichung an Diabetiker, die mit Insulin od.dgl. Mitteln gegen Diabetes behandelt werden, die Stoffwechsellage stabilisiert (normalisiert) und die periphere und zentrale Kapillar-Durchblutung fördert. Die vorerwähnten Probleme, von welchen Langzeitdiabetiker bis nun betroffen waren, können vermieden bzw. auf ein Minimum reduziert werden, so daß ohne großen Aufwand normoglykämische Zustände wieder hergestellt werden können.

Zusätzlich ist es möglich, bereits eingetretene Schäden, je nach deren Grad, teilweise oder sogar zur Gänze zu beheben. Weiters kann der Insulinbedarf auf ein Minimum reduziert und stabilisiert werden, da die Antikörper-Produktion gegen Insulin unterdrückt wird. Zusätzlich ist es möglich, einen bereits erhöhten Insulinbedarf durch Anwendung des durch die Erfindung geschaffenen Heilmittels wieder auf den anfänglichen Insulinbedarf, zum Teil sogar unter denselben, zu senken.

Die Wirksamkeit des durch die erfindungsgemäße Maßnahme geschaffenen Heilmittels dürfte in einer Wechselwirkung zwischen den Stoffwechselkreisen und dem erwähnten Stoffgemisch gelegen sein. Die Stabilisierung (Normalisierung) aller Regelkreise erlaubt durch eine erhöhte Insulin- und Diättoleranz eine problemlose Anwendung der konventionellen Therapie (nur Depotinsulin).

Quercetin kommt in der Natur in den Blüten des Goldlacks, des Löwenmauls und der Rose sowie im Tee und im Hopfen vor.

Es wurde dabei auch gefunden, daß durch das erfindungsgemäß hergestellte Arzneimittel nicht nur eine Stabilisierung, sondern auch eine Normalisierung der Stoffwechsellage auch beim Fettstoffwechsel und auch beim Eiweißstoffwechsel erzielt werden kann. Dies deshalb, weil insbesondere auch durch die Wirkung des Khellins auf low density lipids und high density lipids nicht nur der Zuckerstoffwechsel, sondern vor allem auch der Fettstoffwechsel beeinflußbar und regulierbar ist.

Aufgrund der die Regelkreise (auch hormonelle) und damit die Durchblutung der Gefäße normalisierenden Wirkung des erfindungsgemäß hergestellten Heilmittels werden auch andere Nebenwirkungen, wie z.B. die beim Diabetiker oft auftretende erektile Impotenz, behebbar.

Weiters kann auch durch das erfindungsgemäß hergestellte Mittel aufgrund der Normalisierung des Stoffwechsels eine Gewichtsnormalisierung erzielt werden, d.h., daß aufgrund von Stoffwechselerkrankungen übergewichtige Patienten bei Beibehaltung ihrer Lebensgewohnheiten auf ihr Normalgewicht langsam zurückgeführt werden. Bei Diabetikern kann es dazu führen, daß untergewichtige Diabetiker wieder ihr Normalgewicht erreichen, ohne daß dabei die Insulingabe entsprechend geändert werden muß.

Da die Regelkreise des Körpers durch das erfindungsgemäß hergestellte Mittel in geordnete Bahnen gelenkt werden, können auch Störungen des Abwehrsystems erkannt und wieder mit dem richtigen "Schlüssel" versehen werden und damit normalisiert werden. Es funktionieren dann somit alle Regelkreise des Körpers, und zwar sowohl jene des Kohlenhydratstoffwechsels als auch des Lipid- und des Eiweißstoffwechsels. Es wird also eine Antikörperbildung gegen Insulin der eigenen Betazellen und gegen Fremdinsulin vermieden bzw. unterdrückt. Weiters wird eine Störung der Alphazellen (Glukagonproduktionszellen) unterdrückt, wodurch Schockhypos vermieden werden.

Aufgrund der Normalisierung der Stoffwechsellage des gesamten Organismus wird damit auch die Abwehr gegen Fremdkörper und sonstige Krankheiten erhöht.

### BEISPIEL 1:

Aus Erythroltetranitrat, Phenyläthylbarbitursäure, Papaverin, Belladonnaextrakt, Theobromin und Khellin (5,8-Dimethoxy-2-methylfuro-[4',5':6,7]-chromon) wurden Dragees hergestellt, von welchen jedes 0,003 g Erythroltetranitrat, 0,02 g Phenyläthylbarbitursäure, 0,04 g Papaverin, 0,003 g Belladonnaextrakt, 0,15 g Theobromin und 0,0025 g 5,8-dimethoxy-2-methylfuro-/4',5':6,7/-chromon enthielt. Eingenommen wurden (zusätzlich zur Insulinbehandlung) täglich 3 - 4 Dragees, in gleichmäßigen Zeitabständen. Es ergab sich eine Reduzierung des Insulinbedarfes von 85 Eh auf ca. 50 Eh, wobei 50 Eh die Anfangstagesdosis am Beginn einer ca. 30jährigen Insulinabhängigkeit betrug. Es konnte sowohl eine normale Funktion der Nieren als auch eine einwandfreie Coronardurchblutung beobachtet werden. Ebenso blieb die Schmerz-, Kälte- und Hitzewahrnehmung sowie die normale Durchblutung in den Extremitäten erhalten. Auch im Sehvermögen trat - über die altersbedingte Abnahme hinaus - keine Verschlechterung ein. Im Denk- und Wahrnehmungsvermögen trat nach der Behandlung durch Einnahme der, wie vorstehend beschrieben zusammengesetzten, Dragees eine deutliche und anhaltende Besserung ein.

Anstelle von Dragees können auch Pulver oder Tabletten, welche die gleiche Rezeptur aufweisen, verabreicht werden.

In obigem Beispiel kann Erythroltetranitrat ersetzt werden durch Isosorbitdinitrat. Isosorbitmononitrat, Nitroglycerin oder Nitropentaerythrolum bei etwa gleicher Dosierung, was auch für einen etwaigen Ersatz von Khellin (5,8-Dimethoxy-2-methylfuro-[4',5':6,7]-chromon) durch Pentifylin, Cinnerizin, Xantinolnicotinat oder durch Extr.Ginkgo bil. bzw. Quercetin gilt. Der Anteil der Mittel zur Förderung der peripheren und der Mittel zur Förderung der zentralen Durchblutung richtet sich nach den Erfordernissen, die durch den Zustand des Patienten gegeben sind.

Nachstehend wird ein Genesungsverlauf eines Diabetikers sowie ein Genesungsverlauf einer übergewichtigen Person, welche das übergewicht aufgrund einer Stoffwechselerkrankung besitzt, beschrieben. Aus diesen Genesungsverläufen ist die Wirkung des erfindungsgemäß hergestellten Mittels erkennbar.

### BEISPIEL 2:

### Diabetiker:

- Augen:: 10 Wochen nach Behandlungsbeginn bildet sich eine bereits vorhandene Retinopathie, welche vom Spezialisten an der Universitätsaugenklinik bescheinigt worden war und welche mit Laserstrahltherapie behandelt hätte werden sollen, zurück. Das Sehvermögen nimmt zu. Nach weiteren 30 Wochen Behandlung ist Retinopathie nicht mehr vorhanden, das Sehvermögen wird wieder so gut, daß bei guter Beleuchtung die Zeitung wieder ohne Lesebrille (Alter 58 Jahre!) gelesen werden kann. Die Lesebrille wird nur noch bei schlechter Beleuchtung zum Lesen verwendet.
- Nieren:: 10 Wochen nach Behandlungsbeginn tritt die erste Besserung der bereits eingetretenen Nephropathie ein, die Beschwerden nehmen ab. Nach weiteren 20 Wochen Behandlung verschwindet die Nephropathie ganz; das Wohlbefinden ist wieder hergestellt.
- Gehirn:: 16 Wochen nach Behandlungsbeginn nimmt die Konzentrationsfährigkeit langsam zu, das bereits debile Verhalten wird langsam weniger debil, das Gedächtnis kehrt in ersten Anfängen wieder zurück. Logische Denkfehler werden bereits geringer und das kindisch-senile Verhalten tritt etwas in den Hintergrund, ebenso die totale Antriebsschwäche. Nach weiteren 52 Wochen Behandlung kommt die Konzentrationsfähigkeit wieder voll zurück. Das Verhalten des Patienten wird wieder normal, das Gedächtnis setzt wieder voll ein, das logische Denken ist wieder vorhanden. Ebenso ist die alte Antriebskraft wieder voll vorhanden, das Tempo der geistigen und körperlichen Handlungen ist zurückgekehrt. Das Sprechen ist wieder fließend und logisch (Die unvollendeten Halbsätze gehören wieder der Vergangenheit an).

### BEISPIEL 3:

- Gewichtsabnahme eines Nichtdiabetikers:: Nach 2 Wochen Behandlung sank das Körpergewicht von 105 auf 103 kg (Körpergröße 1,68 m, Alter 51), nach 4 Wochen auf 101 kg, nach 6 Wochen auf 99 kg, nach 8 Wochen auf 97 kg, nach 11 Wochen auf 96 kg, nach 14 Wochen auf 95 kg, nach 17 Wochen auf 94 kg, nach 19 Wochen auf 93 kg, nach 21 Wochen auf 92 kg. Dabei hat der Patient die Lebensweise und die Ernährung nicht geändert. Der Patient spürte, wie die in den letzten Jahren ständig angesetzten Fettzellen an Armen, Beinen, Gesicht, Rücken, Hüften weniger wurden. Die ödeme im Gesicht, unter den Augen verschwanden.

## Patentansprüche

1. Verwendung mindestens eines Stoffes, ausgewählt aus Phenyläthylbarbitursäure, Papaverin, Belladonnaextrakt und Theobromin im Gemisch mit einem Mittel zur Förderung der peripheren Durchblutung, z.B. Khellin (5,8-Dimethoxy-2-methylfuro-[4',5':6,7]-chromon), Khellinabkömmlinge, Pentifylin, Cinnerizin, Xantinolnicotinat oder Extr.Ginkgo bil. bzw. Quercetin (3.5.7.3'.4' Pentaoxyflavon) und/oder eines Mittels zur Förderung der zentralen Durchblutung, z.B. Erythroltetranitrat, Isosorbitdinitrat, Isosorbitmononitrat, Nitroglycerin oder Nitropentaerythrolum zur Herstellung eines Arzneimittels zur Stabilisierung bzw. Normalisierung des Stoffwechsels und/oder des Immunsystems von Patienten mit Stoffwechselerkrankungen, wie z.B. von Diabetikern, insbesondere von Insulinpflichtigen.

2. Verwendung des in Anspruch 1 genannten Stoffgemisches zur Herstellung eines Arzneimittels zur Normalisierung der hormonellen Regelkreise, zur Verringerung bzw. Vermeidung der Antikörperbildung gegen in Diabetes-Mitteln, z.B. Insulin, enthaltenes körperfremdes Eiweiß und gegen körpereigenes Insulin und dadurch zur Erhöhung deren Akzeptanz, sowie gegen eine Störung der Alphazellen (Glukagonproduktionszellen).

## Claims

1. Use of at least one substance, selected from phenylethylbarbituric acid, papaverine, belladonna extract and theobromine in a mixture with an agent aimed at promoting peripheral blood circulation, e.g. khellin (5, 8-dimethoxy-2-methylfuro- [ 4', 5': 6,7]-chromone), khellin derivatives, pentifylin, cinnarizine, xanthinol nicotinate or extr. gingko bil or quercetin (3.5.7.3'.4' pentaoxyflavon) and /or an agent for promoting central blood circulation, e.g. erythrol tetranitrate, isosorbite dinitrate, isosorbite mononitrate, nitroglycerin or nitropenta-erythrolum, for the manufacture of a drug for stabilising or normalising metabolism and/or the immune system of patients with metabolic diseases, such as for example diabetics, in particular insulin-dependent patients.

2. Use of the mixture specified under Claim 1 for the manufacture of a drug for normalising the hormonal control systems, for reducing or preventing antibody formation against exogenous protein contained in diabetes remedies, e.g. insulin, and against endogenous insulin, and therefore for increasing their acceptance, as well as against disorders of alpha cells (glucagon production cells.)

## Revendications

1. Utilisation d'une substance au moins soit, au choix, acide barbiturique phényléthylique, papavérine, extrait de belladone et théobromine, mélangée à une substance stimulant la circulation sanguine périphérique, par exemple khelline (5,8-diméthoxy-2-méthylfuro-[4',5':6,7]-chromone), dérivés de khelline, pentifyline, cinnerizine, xantinolnicotinate ou extrait de gingko bil. ou quercétine (3.5.7.3'.4' pentaoxyflavone) et/ou une substance stimulant la circulation sanguine centrale, par ex. érythroltétranitrate, isosorbitdinitrate, isosorbitmononitrate, nitroglycérine ou nitrapentaérytrolum pour la fabrication d'une drogue permettant de stabiliser ou de normaliser le métabolisme et/ou le système immunitaire des patients souffrant de maladies du métabolisme, comme par exemple les diabétiques, notamment ceux qui sont sous insuline.

2. Utilisation du mélange de substances mentionné à la demande 1 pour la fabrication d'une drogue permettant de normaliser les circuits de régulation hormonaux, de réduire ou d'éviter la formation d'anticorps contre les protéines exogènes contenues dans les drogues contre le diabète, par ex. l'insuline, et contre l'insuline de l'organisme, et permettant donc d'accroître leur acceptance, ainsi que contre un desordre des cellules alpha (cellules de production du glucagon).
